# EUROPEAN PATENT APPLICATION

(11) **EP 4 678 149 A1**
(43) Date of publication of application: **14.01.2026**
(21) Application number: 24187220.9
(22) Date of filing: 08.07.2024
(51) Int. Cl.: A61F 13/00, A61F 13/0246, A61F 13/02

(54) **IMPROVED WOUND DRESSINGS AND INTEGRATED PROCESS FOR THE MANUFACTURE OF WOUND DRESSINGS**

(71) Applicant: Mölnlycke Health Care AB, 431 21 Mölndal (SE)
(72) Inventor: JOHANNISON, Ulf, 438 91 Landvetter (SE)
(74) Representative: Tostmann, Holger Carl

(57) **Abstract**

The present invention relates to an improved integrated process for the continuous manufacture of wound dressings, in particular so-called "island dressings", i.e., dressings that comprise a laminate of layers of different functionalities including (but not limited to) a backing layer, an absorbent pad, a support layer, and a skin or wound facing adhesive layer comprising a silicone gel.

The integrated process of the invention limits or minimizes the use of resources, components, materials or processing aids and/or energy vis-à-vis processes known from the art. In particular, the process of the invention minimizes or avoids the use of "constructive" adhesives that hold different layers of a dressing together and results in a wound dressing with superior performance properties.

## Description

### Field of the Invention

The present invention relates to an improved integrated process for the continuous manufacture of wound dressings, in particular so-called "island dressings", i.e., dressings that comprise a laminate of layers of different functionalities including (but not limited to) a backing layer, an absorbent pad, a support layer, and a skin or wound facing adhesive layer comprising a silicone gel. The integrated process of the invention limits or minimizes the use of resources, components, processing aids or materials and/or energy vis-à-vis processes known from the art.

In particular, the process of the invention minimizes the use of "constructive" adhesives that hold different layers of a dressing together and results in a wound dressing with superior performance properties.

### Background of the Invention

In the area of treatment of wounds, it is generally undesirable that the layers of a wound dressing come apart, when the wound dressing is applied to a wound or when the dressing is removed from a wound. Therefore, wound dressings of the art comprising individual layers of different functionality being laminated together are typically strongly bonded together by using a "constructive" adhesive.

In particular, the presence of "constructive" adhesive layers between layers of a wound dressing laminate may impair fluid transportation within in the wound dressing laminate and the moisture vapor transmission rate of the backing layer, thus impairing the overall liquid handling capacity of the dressing.

Furthermore, the presence of an adhesive layer between backing layer and absorbent pad may lead to a potentially undesirable increased stiffness of the dressing, which may present a hindrance to tightly applying a dressing to non-planar skin or wound topographies.

According to US 7 619 130, if the backing layer is coated with adhesive, the permeability of the backing layer will decrease. US 5 973 22 discloses both the use of an adhesive or the use of heat bonding to adhere a backing layer to a foam layer and points to the potential drawback of "interference" of an adhesive with moisture vapor permeability.

In another important aspect to be considered for any process for the manufacture of wound dressings is to minimize the use of materials and energy in the overall process. Generally, in processes known from the prior art for the manufacture of wound dressings, for example from US 7 745 682, the overall process relies on sacrificial layers, for example carrier layers having removable paper thereon, which must be discarded after use. Depending on the supporting layers used in the overall process, delamination of those parts of the wound dressing that are to be further processed can also become an issue.

In continuous processes known from the art, "in-line" control of the dressings (which are only available for testing as actual dressings at the very end of the process) is difficult to implement and it would be desirable to conduct the process for manufacture so that key properties of a dressing can be controlled (and the process accordingly adjusted as necessary) while the process is running, for example while a laminate is continuously moving forward in a process.

Based on the discussion of the potential drawbacks known from the processes and products of the prior art, it is an objective of the present invention to provide an integrated process for the continuous manufacture of wound dressings, in particular island dressings, and to provide a wound dressing, that avoids or minimizes all or some of the drawbacks discussed above.

In particular, an r object of the present invention is to provide an overall integrated process for the continuous manufacture of wound dressings, in particular island dressings, that minimizes the use of material, in particular the use of sacrificial layers that need to be delaminated and discarded, but also minimizes the need for separate components in the manufacturing equipment and/or minimizing the use of materials and/or energy required.

A further object of the present invention is to provide an overall integrated process for the continuous manufacture of wound dressings, in particular island dressings, wherein in-line process control of performance parameters of a dressing is possible.

### Brief Description of the Figures:

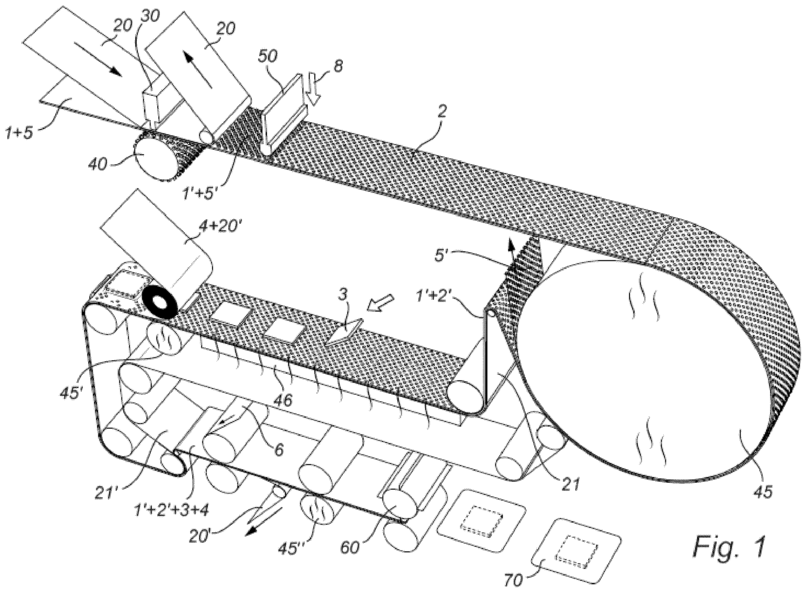

**Figure 1** provides an exemplary overview of aspects of an integrated and continuous process for the manufacture of island dressings, beginning with the feeding of the structural layer (1) and process aid layers and ending with the cutting of the resulting laminate into individual island dressings (70).

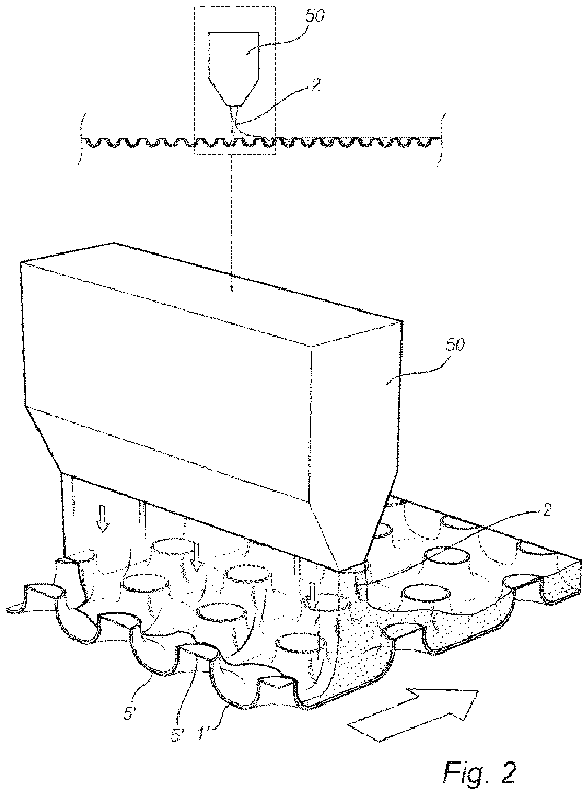

**Figure 2** highlights a further exemplary but more detailed aspect of the overall process, namely the step of dispensing an uncured silicone mixture onto a perforated support layer (1'), wherein the perforations in the support layer are "protected" by a deformed template layer (5') that prevents the silicone mixture from entering into or covering the perforations in the support layer (1'). In case "slot" dispensing is performed, as shown in Figure 2, initially, the entirety of the laminate is covered by silicone mixture.

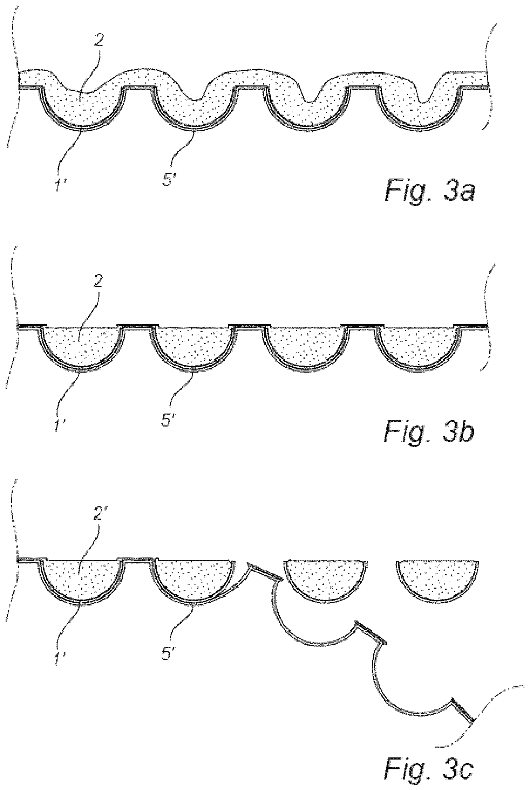

**Figure 3** shows a cross-section of an exemplary laminate resulting after the silicone mixture dispensing step (B): The upper panel (Fig. 3a) shows a cross-section of the layers immediately after dispensing: the silicone mixture (2) "evenly" covers the entire deformed template layer (5'). The middle panel (Fig. 3b) shows the same cross-section after the coated laminate (1') + (2) + (5') has traveled along the moving line [see Figure 1: relevant is the part of the moving line prior to the curing step on the heated rotating drum (45)]; by then, the silicone mixture (2) has mostly flown into the "valleys" provided between the protruding parts of the deformed template layer (5'); the top parts of the deformed template layers are only covered by a thin film of the silicone mixture. The lower panel (Fig. 3c) shows a cross-section of the product resulting from the delamination step [see also Figure 1: removal of (5') from (1') + (2') immediately after the laminate leaves the heated rotating drum (45)]: the deformed template layer (5') is removed together with a thin silicone gel layer (now at least partially cured) and the remaining laminate comprises at least partially cured silicone gel (2') with opening essentially aligning with the perforations of the support layer (1').

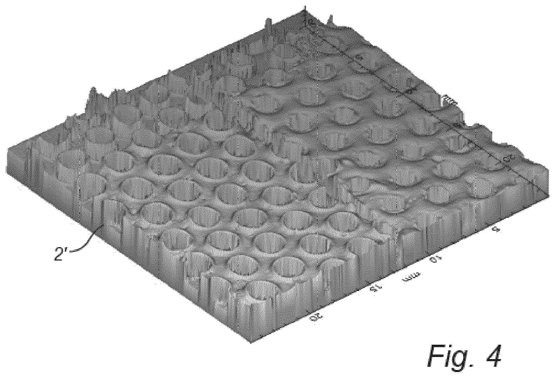

**Figure 4** shows a surface scan of a fully cured silicone gel, wherein the segment from 12 mm to 30 mm shows a cured silicone gel layer that has been perforated by perforating elements (sample made according to the prior art) while the segment from 0 mm to 12 mm shows the openings obtained in accordance with steps (A) and (B) of the present invention (sample with no "donut"-shaped elevations around the opening). For sake of comparison surface scans of the two different samples are shown immediately next to each other.

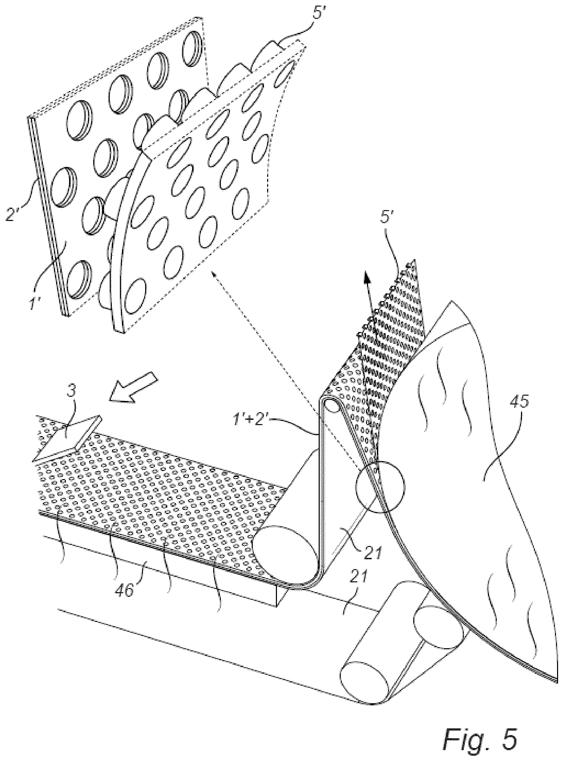

**Figure 5** highlights a further exemplary but more detailed aspect of the overall process, namely the transferring step (D): while the deformed template layer that is no longer needed after the curing step (C) is removed from the continuous process, the cured silicone gel layer on the support layer (both having aligned perforations/openings) is transferred onto a moving (Teflon) belt. This Figure shows a perspective corresponding to the cross-sectional view in Figure 3c.

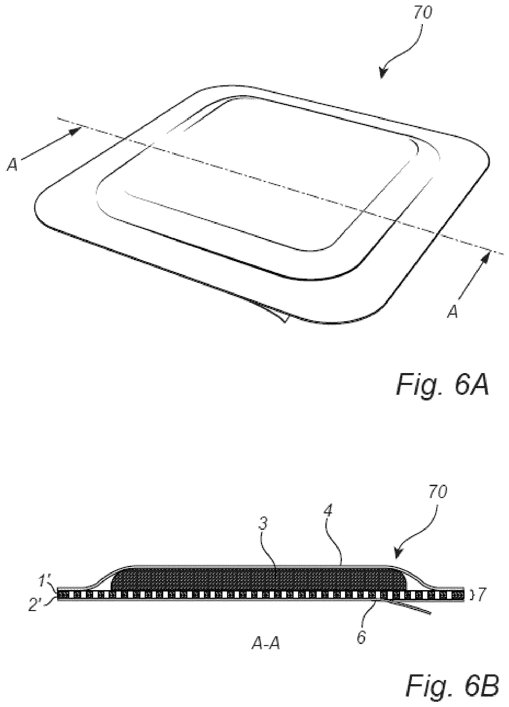

**Figure 6** shows an exemplary embodiment of an island dressing (70) that may be produced using the disclosed process, with the following sequence of layers, starting from the side of the dressing intended to be applied onto the wound: release liner (6) (to be removed prior to use) / silicone gel layer (2') with openings aligning with the perforations in the support layer / perforated support layer (1') / absorbent pad (3) / backing layer (4).

### Summary of the Invention

The above-mentioned objects, and others, are solved by the following continuous process for the manufacture of wound dressings, in particular island dressings, wherein said process at least comprises a step of providing laminate as defined below, at least followed by an absorbent pads application and laminating step (E) and a backing layer laminating step (F):
Providing a laminate on a moving belt, wherein said laminate comprises at least the following layers in the following sequence of layers, starting with the layer that is in direct physical contact with said moving belt: a layer of silicone gel that has an array of openings and a perforated support layer that has an array of perforations that align with said array of openings in the silicone gel layer;
said process also comprising at least the following further steps (E) and (F):
   (E) continuously applying absorbent pads onto said laminate comprising at least a perforated support layer and a silicone gel layer, wherein the absorbent pads are applied onto the side of the support layer that is not coated with silicone gel, wherein said absorbent pads are at least partially heat bonded to said side of the support layer not coated with silicone gel;
   (F) continuously feeding a backing layer onto the laminate from step (E), wherein the backing layer is applied onto the absorbent pads and the side of the support layer that is not coated with silicone gel, wherein the backing layer is at least partially heat bonded to said side of the support layer that is not coated with silicone gel.

The application step (E) is illustrated in Figure 1, central part: absorbent pads (3) are applied onto the laminate with the perforated support layer facing the direction of application pads (3).

In preferred embodiments, the entire laminate comprising at least (1') + (2') and sitting on top of the moving belt (2) is heated from below along the heating zone (46) [ "flame"-symbols indicate contact heating)]

In embodiments, the at least partial heat bonding of the absorbent pads to the support layer is achieved by heating the moving belt from below so that the temperature of the support layer is in the range of from 100°C to 180°C, preferably from 120°C to 160°C.

Without wishing to be bound by theory, it is believed that if the moving belt is heated from underneath, i.e. from the side that is not in direct physical contact with the laminate, to a temperature in the range of from 100°C to 180°C, preferably of 120°C to 160°C, the support layer is sufficiently softened that at least a partial heat lamination, preferably full heat lamination, occurs between at least parts of the absorbent pads and the support layer, for example by way of "physical" bonding between the two surfaces (lower surface of the backing layer, upper surface of the support layer).

In a subsequent or concurrent laminating step, a backing layer is continuously applied onto the laminate including the absorbent pads from the previous application step (E) and the backing layer is at least partially heat bonded at least to the support layer:
This laminating backing layer step is illustrated in Figure 1, lower left part of the Figure: a backing layer (4) [together with a processing aid layer (20')] is continuously applied onto the laminate comprising perforated support layer (1'), silicone gel layer (2'), wherein absorbent pads are included in this laminate. The laminate continuously moves as transported by the moving belt (21). In this exemplary embodiment, laminate, backing layer and belt are all fed together and pressed together between two rolls, wherein the lower roll (45') is heated (see "flame" symbols). The processing aid layer (20') is removed further down the line [after application of the release liner (6); see lower left part in Figure 1]. The purpose of this processing aid layer is to support and stabilize the comparatively flexible backing layer.

In embodiments, the at least partial heat bonding of the backing layer to the support layer is achieved by heating the moving belt, or a lower roll, or both, to a temperature in the range of from 100°C to 180°C, preferably of 120°C to 160°C.

Without wishing to be bound by theory, it is believed the support layer is sufficiently softened by this heating so that at least a partial heat lamination, preferably a full heat lamination, occurs between at least parts of the backing layer and the support layer by way of "physical" bonding between the two surfaces (lower surface of the backing layer, upper surface of the support layer).

In embodiments, the at least partial heat bonding of the backing layer onto the support layer is also/ further facilitated by exerting pressure onto the laminate comprising the backing layer and the support layer, preferably by way of pressing the laminate between an upper roll and a lower roll (for an illustration see discussion of Figure 1 above).

In preferred embodiments, exerting pressure onto the laminate also comprising the absorbent pads, preferably by way of pressing the laminate between an upper roll and a lower roll, further facilitates the at least partial heat bonding between the support layer and the absorbent pads.

In embodiments, the upper roll is softer than the lower roll.

In embodiments of the invention, the lower roll underneath the laminate comprising the silicone gel / perforated support layer / absorbent pad is heated to a temperature in the range of from 100°C to 180°C, preferably from 120°C to 160°C

Step (F) of heat bonding the backing layer onto the support layer may be performed at least partly *concurrently* with step (E) of heat bonding the absorbent pads onto the support layer.

In embodiments, the temperature of the lower roll underneath the moving belt, and/or the speed of rotation of the lower roll and/or the pressure exerted by pressing the laminate between said upper and said lower roll is/are adjusted so that the backing layer and the absorbent pads are at least partially heat bonded (laminated) onto the support layer, optionally so that the backing layer is also at least partially heat bonded onto the absorbent pads.

The two steps (E) and (F) involving heat bonding are illustrated in Figure 1: The absorbent pads (3) are applied onto the perforated support layer (2') while the moving belt is heated from underneath in the heating zone (46). Full or partial heat bonding (= laminating) of the absorbent pads onto the support layer is achieved in that heating zone.

Further heat bonding of the absorbent pads onto the support layer may be achieved in the subsequent step of adding the backing layer (6) wherein the entire laminate, including the absorbent pads, is pressed between two rotating rolls, wherein at least the lower roll (45') is heated. Heat and/or pressure achieve heat bonding/lamination of the backing layer (4) onto the perforated support layer (1'). Optionally and to a varying degree (depending on temperature, pressure etc.), heat bonding/lamination of the backing layer onto the absorbent pad and/or of the absorbent pad onto the support layer may also be achieved in this step.

As a result of these laminating/ heat bonding steps, the resulting laminate as transported on the moving belt has the following sequence of layers beginning with the layer that is in physical contact with the moving belt: silicone gel with openings that essentially correspond to the perforations of the support layer / perforated support layer / absorbent pads / backing layer covering the absorbent pads and the outwardly facing side of the support layer in a border region.

As a result of step (F), the backing layer is at least partly heat bonded to the support layer in the border region around the absorbent pad, i.e. the area that does not comprise an absorbent pad. In the final product (wound dressing), the absorbent pad has a smaller area than the backing layer and the support layer, if viewed from top or form below (see Figure 6).

As is the case for all embodiments of the present disclosure, if a specific sequence of layers is disclosed, any other number or kind of layers may be present at any position, as long as the sequence of the specifically mentioned layers is maintained.

For example, a further layer may be present between support layer and absorbent pad/backing layer. In particular, the absorbent pad may include several sublayers (e.g., the absorbent pad may be a laminate of a foam layer and a fibrous layer).

In embodiments, the backing layer comprises or consists of a polyurethane polymer, in particular a film of a melt-blown polyurethane polymer, which is optionally fed together with a further supporting layer in step (F), which further supporting layer preferably comprises or consists of polyethylene.

Without wishing to be bound by theory, the heat provided by the rotating roll underneath the moving belt (and therefore also underneath the laminate) is sufficient to soften the materials in question, in particular the support layer and possibly, at least to some extent, also the backing layer so that it is possible to partially or fully heat-bond the backing layer onto the support layer.

In preferred embodiments, the upper roll is made of a comparatively soft material, for example a polymeric material, whereas the lower roll is made of a comparatively solid material, for example a heat-conducting metal.

It is also within the scope of the present invention that in step (F), the backing layer is also at least partly heat bonded onto the absorbent pads. Advantageously, the upper roll is also heated in that case.

The fact that a moving belt is used in the overall process is advantageous for any process step that involves heat bonding/laminating, such as step (E) or step (F), since the moving belt facilitates an even transfer of heat from below, for example from a heated roll to the parts of the laminate that are to be bonded together.

The fact that the backing layer and the support layer are at least partially heat-bonded with each other, as well as that the absorbent pad is at least partly heat bonded onto the support layer is associated with the advantage that no construction adhesive is needed to laminate those layers together in the boundary area around the wound pads.

Any introduction of a further layer, in particular a layer of an adhesive creates a hindrance to fluid flow, be it a wound exudate that is to be transported away from the wound, or be it vapor that needs to be transported out of the wound dressing by way of evaporation through a vapor-permeable backing layer.

In preferred embodiments of the present process, any of steps (A) through (D) as described below, preferably all of steps (A) through (D) precede steps (E) and (F):
(A) **[feeding and perforation step]** feeding at least a template layer and a support layer through an ultrasonic perforation device,
   which ultrasonic perforation device comprises a plurality of perforation elements, preferably an array of perforation elements, and which device is configured to introduce perforations, in particular an array of perforations, into at least said support layer;
   wherein the perforating elements perforate the support layer, while, at the same time, deform the template layer without perforating the same;
(B) **[silicone mixture dispensing step]** after completion of step (A), dispensing a silicone mixture onto the support layer so that the silicone mixture at least covers the non-perforated parts of the perforated support layer as present on top of the deformed template layer.

In accordance with the present disclosure, any layer is suitable as a "support" layer as long as the layer is capable to support the silicone mixture and then the cured silicone gel throughout the entire process of manufacture (see Figure 1) and throughout the use of the dressing.

In accordance with the present disclosure, a silicone "mixture" comprises at least two components that have been mixed prior to dispensing, or are mixed in the dispensing step, wherein the mixture has not been curing at all or only just starts the curing process once mixed.

By contrast in accordance with the present disclosure, a silicone "gel" has already at least partially cured, i.e. if a fully cured silicone gel has 1^00% cured, a partially cured silicone gel is cured at least 30%, preferably at least 50%, further preferably at least 60%.

The specific degree of curing is of no relevance as long as the silicone mixture as dispensed in step (B) has not already partially cured when dispensed, while the silicone gel after step (C) should be cured to a degree that the skilled person understands will be suitable for the intended use.

Curing times and temperatures that are suitable to reach a desired degree of curing, commensurable with the intended use, are different from silicone mixture to silicone mixture but generally known to the skilled person. The degree of cuing can be determined, for example, by rheometric measurements or by near IR measurements.

In particular, the support layer suitably has a melting or softening temperature or range that allows the layer to stay intact and functional during any process step involving energy (heat) intake.

Furthermore, the support layer should be of sufficient flexibility and thickness, among others, so that the resulting wound dressing is suitable for the intended use. The support layer is not only important during manufacture of the dressing but is also an integral part of the dressing at the point of use.

In aspects, the support layer comprises a polyurethane polymer or is a layer of a polyurethane polymer.

In aspects the support layer as fed to the ultrasonic perforation device comprises a layer of a polyurethane polymer together with a layer of a polyethylene polymer.

In specific aspects, a polyethylene polymer layer supports the comparatively thin polyurethane layer and functions as a processing aid, i.e. is removed in the process and does not become a part of the dressing.

In accordance with the present disclosure, any layer is suitable as a "template" layer as long as the layer can be deformed by perforation elements in an ultrasonic perforation process/device.

The template layer should preferably be adapted to maintain its physical shape after deformation and in subsequent process step(s). Once the template layer is delaminated from the laminate, the template layer plays no further role in the dressing.

In aspects, the template layer comprises a thermoplastic polymer that has a higher melting point, by at least 10 K, preferably by at least 20 K, than the polymer(s) making up the support layer.

In aspects, the template layer comprises polypropylene (PP) polymer or is a layer of a polypropylene (PP) polymer.

In preferred embodiments, the overall process is continuous.

In aspects, the template layer and the support layer are fed continuously in step (A).

In the meaning of the present disclosure, "continuous" (feeding) refers to the realization of a process wherein a laminate is continuously moved through the production line/ process steps, and wherein the laminate is successively built up to ultimately result in a continuously moving laminate comprising all layers required for the final dressing. The final individual dressings may then be obtained, for example, by cutting individual dressings out of a continuously moving laminate (see the rotating die cutter device in Figure 5).

In preferred embodiments, the overall process is operated continuously and is preferably operated so that at least 100 dressings, preferably at least 1000 individual dressings result from the overall process

In the meaning of the present disclosure, a "laminate" refers to two or more sheets or layers (these terms are used interchangeably and synonymously) that are (at least lightly) bonded together, for example by feeding one layer over another layer under pressure and/or shear, and/or by heat bonding two or more layers together.

Dispensing an (uncured) silicone mixture onto another layer and subsequently curing the same also results in a "laminate" in the meaning of the present disclosure.

Bonding absorbent pads onto another layer also results in a "laminate" in the meaning of the present disclosure.

In embodiments of the present invention, the laminate does not comprise any "constructive" adhesive between any of the functionally different layers of the laminate, i.e. any adhesive that has the function to adhere one layer onto another layer,

In preferred embodiments, the laminate does not comprise any "constructive" adhesive between the backing layer and the support layer.

A layer of silicone gel as possibly present in the wound contact layer does not have the function to adhere one layer onto another layer but rather has the function to adhere the overall dressing to a patient, during use.

As is customary for wound dressings, the wound contacting *outer* adhesive layer (for example a silicone gel layer) may be protected by a release liner, which is then part of the "laminate" (irrespective of the fact that the release liner is removed prior to use while the other layers of the laminate remain adhered to each other during use and also upon removal of the laminate after use). The presence of a release liner that is adhered to the silicone gel prior to use of the dressing also does not render the silicone gel a "constructive adhesive" as used as a term throughout this disclosure.

In the meaning of the present disclosure, "deforming" a layer, in particular deforming a template layer refers to the process step of changing the shape of the layer in a way that the changed or "deformed" shape persists, even if the elements that have caused the deformation (here: the perforation elements) have been removed from the deformed layer, i.e., are no longer in contact with the layer. Such a lasting or persisting deformation is suitably achieved with a layer made of or comprising a thermoplastic material, in particular in case energy (heat) is applied, as is the case here (ultrasonic perforation or deformation), resulting in a softening or partial melting of the layer.

In the meaning of the present disclosure, "feeding" at least a template layer and a support layer "through" an ultrasonic perforation device, means that these at least two (or more) layers are continuously moved between two opposing elements of an ultrasonic perforation device, in particular between a sonotrode and an array of perforation elements (see more detailed description of an ultrasonic perforation device below), and are brought into contact with each other at least at the point of contact with the perforation elements.

In aspects (not shown in Figure 1), the perforation elements may be arranged in patterns, for example so that perforations are only present in those parts of the support layer that is in contact with the absorbent pads in the final wound dressing. The functionality of perforations to facilitate uptake of wound exudate for absorption is relevant only (or primarily) for those parts of the wound dressing that actually are in contact ("fluid communication") with an absorbent material. An example of a dressing that has perforations or incisions only or primarily in the area of the wound contact layer that coincides with the absorbent pad can be found, for example, in WO 2016/169948 (see, e.g., Fig. 1) and in WO 2017/081012 (see, e.g. Fig. 3a).

In aspects of the feeding and perforation step (A), the support layer by itself may be fed as a laminate, for example as a laminate of a layer of a polyurethane polymer (which would be the part of this laminate that ultimately remains in the dressing as a support layer for the silicone gel) and a reinforcing layer of a polyethylene (PE) polymer, which may be removed at a subsequent step of the process.

In further aspects, a sacrificial paper layer, preferably a paper layer coated with Teflon, is continuously fed together with the support layer and the template layer. The purpose of the paper layer is to facilitate the perforating/deforming by the ultrasonic device by achieving at least one of the following supporting functions: (i) protecting parts of the ultrasonic device, for example the sonicating horn from melting polymer of the support layer; (ii) improving or more evenly distributing heat uptake; (iii) supporting the laminate during mechanical and temperature stress resulting from the exposure to the perforation elements and the ultrasonic energy intake.

In alternative aspects, instead of a paper film, any suitable (higher melting point) polymer layer may also be used as long as at least one of the supporting functionalities (i-iii) disclosed above is achieved.

An exemplary and schematic sketch of such a preferred line feeding arrangement is shown in Figure 1, which provides an overview over the entire process, from the feeding and perforation step (A) to the final cutting of the laminate into island dressings. Of particular relevance for the feeding and perforation step (A) is the upper left part of Figure 1 showing the co-feeding of support layer (1) and template layer (5), wherein the support layer (1) is perforated between the sonotrode (30) of an ultrasonic perforation device and a roll (40) with perforation elements. As a result of this step, support layer (1') is perforated and template layer (5') is deformed but not perforated. A sacrificial processing aid layer (20), for example a Teflon-coated paper layer as discussed above, may be used.

The silicone mixture dispensing step (B) is also illustrated in the upper left corner of Figure (1) [see silicone mixture dispensing unit (50) and the arrow (8) indicating the dispensing of a silicone mixture (2) onto the perforated support layer (1'). This silicone mixture dispensing step (B) is shown in more detail in Figure 2: a silicone mixture dispensing unit (50) dispenses a non-cured (or only minimally curing) silicone mixture (2) onto the perforated support layer (1') while "surrounding" the parts of the deformed template layer (5') that protrude through the perforations of the support layer (1'). These protrusions of the deformed template layer prevent the silicone mixture from moving into the perforations of the support layer.

As an intermediate product of said perforation step (A), a laminate results that comprises a perforated support layer on top of a deformed (but not perforated) template layer, wherein the deformations in the template layer consist of elevated portions, wherein the elevated portions of the deformed template layer protrude through the perforations of the support layer. In essence, the perforations in the support layer are substantially "filled out" by these elevated portions of the deformed template layer, thus creating continuous "valley" areas on the surface of the support layer around the elevated portions that protrude the perforations of the support layer (see Figure 2 showing. a schematic depiction of a cross-section of an exemplary laminate resulting from the ultrasonic perforation step).

In aspects, the silicone mixture in dispensing step (B) is dispensed as an uncured or only partially curing silicone mixture, for example as a partially curing two-component silicone mixture, wherein the two silicone components preferably are mixed for the first time in the dispensing unit (prior to this mixing, no curing is possible).

Exemplary and preferred silicone mixtures to be applied in dispensing step (B) are described in more detail below.

In the meaning of the present invention, the silicone mixture is a layer that is on "top" of the support layer in the meaning that during the dispensing step, in the direction of the gravitational force, the support layer is underneath the silicone mixture as dispensed. During use and once the silicone mixture has cured [i.e., after step (C)], the dressing may be inverted so that the silicone gel may be underneath the support layer in the direction of gravitation, however, the silicone gel is still "on top" of the support layer in the sense that it is coated (and was dispensed) onto the same.

**Figure 3** shows a cross-section of an exemplary laminate resulting after the silicone mixture dispensing step (B): Figure 3a shows a cross-section of the layers immediately after dispensing: the uncured silicone mixture (2) "evenly" covers the entire deformed template layer (5'). The protruding parts of the deformed template layer (5') "cover" and "protect" the perforations of the support layer (1').

Fig. 3b shows the same cross-section after the coated laminate (1') + (2) + (5') has traveled along the moving line [see Figure 1: relevant is the part of the moving line prior to the curing step on the heated rotating drum (45)]; after moving along the line, for example for 20 seconds, the silicone mixture (2) has mostly flown into the "valleys" provided between the protruding parts of the deformed template layer (5'); the top parts of the deformed template layers are only covered by a thin film of the silicone mixture.

Fig. 3c shows a cross-section of the product resulting from the delamination step, i.e., after curing, for example on a heated rotating drum [see also Figure 1: removal of (5') from (1') + (2') immediately after the laminate leaves the heated rotating drum (45)]: the deformed template layer (5') is removed together with a thin silicone gel layer (now at least partially cured) and the remaining laminate comprises at least partially cured silicone gel (2') with opening essentially aligning with the perforations of the support layer (1').

The protruding parts of the deformed template layer (5') "cover" and "protect" the perforations of the support layer.

Dispensing a silicone mixture, in particular an uncured silicone mixture onto the non-perforated parts of the support layer (while the perforated parts are not coated since these parts are "filled" (or "blocked") by the elevated portions of the deformed template layer), and then curing this mixture of an uncured silicone, results in laminate of a support layer having perforations and a coating or layer of a cured silicone gel that has openings that align with the perforations in the support layer (see Figures 3 and 5).

At the same time, the formation of "elevated" areas ("rings" or "donuts") of a silicone gel around the outer perimeter of the openings/perforations (which typically occurs if the silicone gel is "directly" perforated or otherwise displaced from its original position) is minimized or avoided (see Figure 4 showing a surface scan of a layer of cured silicone gel on a support layer; the left hand side (roughly from 12 mm to 30 mm) shows the surface scan of a sample where perforations on the silicone gel were obtained by prior art ultrasonic perforation, resulting in "donut"-shaped rings around the perforations. A second sample is shown on the right hand side (roughly from 12 mm to 0 mm); here, only the support layer is perforated and the silicone mixture is dispensed as described above (in accordance with the present invention); as is apparent from the juxtaposition of these two different samples, the process according to the present invention allows to obtain a silicone gel surface that has no donut-shaped elevations around the silicone gel. The surface scan was taken with a commercially available FTR surface metrology scanner.

Dispensing (and curing) a silicone mixture onto the non-perforated area of a support layer (while a template layer comprises elevated portions that fill the perforations and create "valley" areas elsewhere) is associated with the advantage that a silicone layer with perforation-like through-holes ("openings") may be created without actually having to perforate or displace the silicone gel.

Since the indentations created in the deformable template layer by way of ultrasonic perforation mimic the shape of the perforating elements, which typically are rod or pin-like, the silicone mixture curing around those indentations will essentially adapt to this shape and thus takes the same shape it would have if it would have been actually perforated by these very same rods or pins.

The process for the manufacture of wound dressings as described herein, preferably comprises a curing step (C) to be implemented after steps (A) and (B):
(C) **[curing step]** at least partially curing the silicone mixture from step (B) to result in an at least partially cured silicone gel.

In aspects, in step (C), the laminate from step (B) at least partially rotates on a heated rotating drum

In aspects, the curing time is determined by (and in turn can be controlled by setting) the temperature of the heated rotating drum, the diameter of the heated rotating drum, and the speed of rotation of the heated rotating drum, among others.

An example of this curing process is shown in Figure 1 [see large heated rotating drum (45) on the right side of Figure 1]: A laminate comprising deformed template layer (5') with the perforated support layer (1') and a silicone mixture (2) coated thereon is heated on a rotating drum (45). In this drawing, as generally applicable to the drawing, "flame" symbols (here: three such flame symbols) indicate heating by contact.

In conventional processes for making wound dressings comprising silicone gels that need to be cured, said curing is typically performed on stationary heated plates and is often performed outside a continuously running system. For example, the laminate comprising the to-be-cured silicone is taken off the continuously running system and transferred onto a separate support layer that is pulled over heated plates.

Furthermore, the present overall process is advantageous in that curing and "perforation" are performed in line and as part of an integrated process. As discussed above, no separate perforation step on an already cured silicone gel is required, but rather the "perforation" happens "automatically" as a result of the dispensing step (B) and the curing step (C).

In aspects, the laminate comprising the to-be-cured silicone mixture, for example a mixture of a two-component system, is continuously cured in-line while the overall processing of the laminate continues (see also Figure 5). Therefore, not only is the overall process indeed integrated and devoid of interruptions or elements of batch processing, but, in addition, separate or different process lines or pieces of equipment such as heating plates or separate oven units is/are avoided (rotating rolls or drums are part of the overall process anyway).

As a further advantage of this aspect, as outlined above, the rotating drum can be flexibly configured to adjust a desired curing time (by way of varying the diameter of the drum, the rotating speed, the temperature of the drum etc.).

The use of a heating drum for curing the silicone gel as is particularly advantageous in a continuous process. In particular, running the laminate with the coated silicone gel on a rotating drum reduces the friction against the heating surface. Close contact to the heated surface can be established by "tensioning" the laminate, for example by the use of sets of rotating rolls pressing against the laminate. The process according to the invention avoids the use of a further sacrificial tissue layer at the curing stage. Such a sacrificial layer or a web is needed in conventional processes in which a polymer laminate coated with silicone gel is moved, on a further support layer, over heated plates.

The curing temperature typically depends on the silicone gel to be cured, for example its composition or the thickness, and on the specific set-up of the rotating drum. Any temperature that suitably cures the silicone gel to a degree so that the silicone gel can be used in a wound dressing is within the scope of the present invention.

Preferred curing temperatures as defined by the temperature on the surface of the heated rotating drum that is in contact with the laminate are from 100°C to 170°C, preferably from 120°C to 150°C.

The curing time typically also depends on the silicone gel to be cured, for example its composition or the thickness, and on the specific set-up of the rotating drum. Any time range that suitably cures the silicone gel to a degree so that the silicone gel can be used in a wound dressing is within the scope of the present invention.

Preferred curing times are in the range of from 20 seconds to 10 minutes, preferably from 20 seconds to 60 seconds.

In aspects, once the curing process on the rotating drum is completed, in accordance with the specifications of the process, the perforated support layer coated with the *cured* silicone gel can be separated from the deformed template layer and, if applicable, from any further reinforcing layer that may have been co-fed with the support layer (and/or template layer) in the feeding and perforating step (A) (see Figure 1).

In preferred aspects, the process also comprises a transferring step (D), after step (C) and preceding step (E) discussed above:
(D) **[transferring step]** separating the perforated support layer with the at least partially cured silicone gel of step (C) from the deformed template layer and, if present, from other optional layer(s), and transferring said separated laminate comprising the at least partially cured silicone layer on the perforated support layer onto a moving belt;

In embodiments, the moving belt is or comprises a composite material that is compatible with temperatures of up to 150°C, preferably up to 200°C (i.e. has high heat resistance) and/or that has a static coefficient of friction ("COF") of below 0.15, preferably below 0.1 (corresponding to lubricated steel)

In preferred embodiments, the moving belt is or comprises a polyfluorinated polymer, preferably polytetrafluorethylene (PTFE or "Teflon"), particularly preferred is or comprises fiberglass reenforced Teflon.

Teflon has COF of 0.04.

In preferred embodiments, said moving belt rotates continuously in the overall system (see Figures 1 and 5).

The transferring step is shown in the central part of Figure 1 and shown in more detail in Figure 3c and in Figure 5: The deformed template layer (5') as tangentially coming off the rotating heated drum (45) is delaminated from the support layer (1') with the at least partially, preferably fully cured silicone gel layer (2'). The deformed template layer may be discarded and is, at any rate, not part of the final dressing. Other processing aid layers may also be delaminated at this stage. The support layer (1') with the at least partially cured silicone gel layer (2') is concurrently transferred onto a moving belt (21). The following process steps are then performed on the laminate as transported onto the moving belt.

As already alluded to above, concurrently with the transfer of the laminate comprising the cured silicone layer on top of the perforated support layer onto the moving belt, which is the laminate that will ultimately end up in the final product (wound dressing), any further layer(s) no longer needed, in particular the deformed template layer, but also other optional layers that may have been introduced previously as processing layers, for example a reinforcing layer for the supporting layer may be removed at that stage.

As a result of this transferring step, the silicone gel is in direct physical contact with the moving belt, preferably a moving belt comprising Teflon, and can be easily removed in a subsequent step further down the line.

The next layer in the sequence of the laminate (on top of the silicone gel layer in contact with the moving belt) is the perforated support layer that has two interfaces, one interface with the silicone gel layer coated thereon and one "outward" facing interface that is capable of receiving the absorbent pads in the subsequent application step (see below).

In aspects, after laminating step (F), a cooling and delamination step (G) is implemented, in which step the laminate resulting from step (F) is cooled down to a temperature range of from 15°C and 40°C, preferably cooled down to a range of from 18°C to 25°C, wherein this cooled laminate is subsequently removed from the moving belt, i.e. delaminated from the moving belt.

After said cooling and delamination step (G), the moving belt, without any laminate deposited thereon, further moves, preferably rotates, in the overall continuous process and - further down the line - is brought into contact with the laminate coming from the heated rotating drum in step (D)

This delamination step is schematically shown in Figure 1 [lower left most corner. Just underneath roll (45')]: the moving (Teflon) belt (21') is separated from the laminate comprising all layers that ultimately end up in the dressing: silicone gel layer (2') on perforated support layer (1'), which is heat laminated with the backing layer (4) and the absorbent pad (3).

Using a pretensioned moving belt and delaminating the laminate from said belt allows for an "inline tackiness control", i.e. allows for measuring the force required for delaminating the silicone gel off of the essentially inert surface of the moving belt. This provides a tangible parameter that allows to evaluate the adhesion force ("tackiness") that the silicone gel will also have on the skin. Based on the results of this inline tack control, portions of the continuously produced laminate can be discarded, if their measured tackiness is not in line with predefined specifications.

A further advantage associated with an integrated and continuous process is that the use of a moving belt, in particular use of a Teflon belt that is reinforced by fiberglass, allows to synchronize process steps over a significant length of the continuously moving belt. For example, since the belt is comparatively strong and stiff, the tension of the belt as applied by the rotating rolls may be adjusted to further improve synchronization of steps and process control.

The materials used to make the moving belt preferably have a low coefficient of friction vis-à-vis other materials, in particular vis-à-vis silicone gel.

Since it is preferred that a laminate comprising silicone gel is transported in lateral direction in a continuous process by way of a moving belt, it is advantageous that it is easily possible to delaminate the overall laminate from the moving belt to implement the final process steps. If a continuously moving belt of a more conventional plastic material were used, for example a conventional somewhat elastic plastic material, not only would synchronization and process control between the different process step be negatively affected, but also a further processing aid, for example a Teflon coated paper would be needed to ensure suitable delamination.

The movement of the belt through steps (D) to (G), or subsets thereof, may be repeated as often as desired.

After step (G), the laminate is subjected to final processing steps that may be implemented advantageously to obtain the desired wound dressings of suitable for use.

In a further optional step (H), a release liner is added onto the outside facing side of the silicone gel as coated onto the supporting layer, i.e. onto the interface that had - up to this point - been in contact with the moving belt.

The step of adding a release line is shown in the lower left part of Figure 1: after separating the laminate from the moving belt, release liner (6) is added to the silicone gel side of the dressing (that has just come off the moving belt) between two counterrotating rolls. Immediately after said step of adding a release liner (and thus covering and protecting the silicone gel layer), the support layer (20') that was added together with the backing layer (4) further upstream (see discussion above) is removed.

An optional (further) laminating of the backing layer to the absorbent pad may be achieved with heated roll (45").

In aspects, the release liner is continuously applied onto the laminate by way of feeding the same between two counterrotating rolls. At this point, in case any further reinforcing layer may still be present, for example a PE layer that has been fed together with the backing layer, is continuously removed resulting in a laminate of the following sequence of layers: release liner / silicone gel / perforated supporting layer / absorbent pad / backing layer.

In an optional further step (I), assuming that heat bonding the backing layer against the absorbent pad is desirable, the backing layer may also be at least partially heat bonded onto the absorbent pad, in particular between two rotating rolls, wherein the lower roll, i.e. the roll in direct contact with the backing layer, is heated to a temperature in the range of from 100°C to 180°C, preferably from 120°C to 160°C (see discussion of Figure 1 above).

Heat bonding the backing layer against the absorbent pad - either as achieved in the laminating step (F) in which the backing layer is heat bonded onto the support layer and/or as achieved in separate step (I) as shown in Figure 1, or both, - is associated with the advantage that a stable direct contact between the backing layer and the absorbent pad facilitates continuous evaporation of water vapor (e.g. from absorbed wound exudate) through the backing layer, which is preferably a moisture vapor-permeable membrane, also and in particular in the wet state during use. Thus, maintaining direct physical contact between the absorbent pad and the backing layer facilitates for the transport of water vapor from the inside of the dressing to the outside of the dressing during use of the dressing.

By providing a heat bond between the backing layer and the absorbent pad such that the absorbent layer is in close or direct physical contact with the backing layer, the overall moisture vapor transmission rate (MVTR), also known as water vapor transmission rate (WVTR), of the dressing may be increased.

On the other hand, for some uses, for example if wound dressings are to be applied to curved areas of the human body, high flexibility of the overall dressing may be desirable and it may not be desirable that the dressing is too stiff, for example by way of providing a strong bonding between the backing layer and the absorbent pad. In such a case, it may be advantageous to not heat bond the backing layer (also) to the absorbent pad.

In embodiments, the backing layer is only partly heat bonded to the absorbent pad, e.g. in a pattern, thus balancing the above discussed functionalities of moisture vapor transmission rate and flexibility.

By using the continuous process of the present invention, which allows to continuously adjust the process parameters, such as the pressure between the rolls, temperature of the rolls, inline speed etc., the heat laminating between the backing layer and the absorbent pad can be continuously adjusted and optimized depending on the desired specifications of the final product.

In a further optional step of the overall integrated process, the laminate from the previous step(s) (G) or (H) or (I) is cut into individual wound dressings comprising a release liner, a silicone gel layer with openings that essentially coincide with the perforations of a perforated support layer, said perforated support layer, an absorbent pad, and a backing layer; preferably the cutting is achieved by means of a rotating die cutter ("RDC" - see (60) in Figure 1). Said rotating die cutter allows to continuously cut individual wound dressings (70) out of a laminate.

A further aspect of the present invention relates to a **wound dressing,** in particular an island wound dressing, said dressing at least comprising:
- at least one absorbent pad;
- a vapor permeable backing layer overlaying said absorbent pad;
- a perforated support layer underneath said absorbent pad, wherein said backing layer and said support layer extend beyond a periphery of said absorbent pad to define a surrounding border portion along said periphery of the absorbent pad;
   further wherein the backing layer and the support layer are at least partially heat bonded together in said border region,
   and wherein the absorbent pad and the support layer are at least partially heat bonded together;
- a silicone gel layer on the side of the support layer that is opposite the side that contacts the backing layer, wherein the silicone gel layer has openings that correspond to the perforations of the perforated support layer.

In preferred embodiments, no adhesive, in particular no acrylic adhesive, is present between said backing layer and said support layer and no adhesive, in particular no acrylic adhesive, is present between said absorbent pad and said support layer.

An exemplary island dressing (70) is shown in Figure 6: Backing layer (4) overlays absorbent pad (3) and is heat laminated to the perforated support layer (1'). Support layer (1') and silicone gel layer (2') together form the wound contact layer (7). Backing layer (4) and wound contact layer (7) form a border portion or border region around the "island" of the absorbent pad (particularly prominently visible in the upper perspective view). The openings of the silicone gel layer (2') align with the perforations of the support layer (1').

In so-called island dressings, this border region may have a width of 1 cm or 2 cm, around the entire circumference of the central absorbent pad.

In embodiments the support layer is perforated only in those parts of the support layer that are in contact with an absorbent pad.

In preferred embodiments, the wound dressing is further characterized in that the dressing overall does not contain any adhesive other than the silicone gel layer, in particular does not comprise any acrylic adhesive.

In embodiments, the thickness of the silicone gel layer does not vary by more than 10%, preferably not by more than 7%, further preferably not by more than 5% across the entire lateral area of the silicone gel layer.

In embodiments, the silicone gel layer with openings has been obtained by applying a silicone mixture onto a perforated support layer and subsequently curing said silicone mixture to result in a at least partially cured, silicone gel,

In preferred embodiments, neither the silicone mixture nor the silicone gel layer has been perforated, in particular neither has been exposed to any perforating elements.

In embodiments, the openings in the silicone gel layer are circular and have a diameter of from 0.5 mm to 2 mm.

In embodiments, the openings in the silicone gel layer are arranged in an array, wherein the center of each opening is spaced apart from a neighboring center of another opening by a distance of from 2 mm to 4 mm.

In embodiments, the backing layer and the support layer are at least partially heat laminated together so that the adhesion between these two layers exceeds the peeling strength required to remove the entire wound dressing from the wound.

As mentioned above, using heat bonding for lamination, in particular avoiding the presence of any "construction" adhesive between the backing layer and the support layer and/or the absorbent pads and the between the support layer and the absorbent pads, in a wound dressing, is associated with several advantages, one of them being that moisture transmission may be improved, another advantage being that less components and materials need to be used in the process and less materials end up in the product.

More specifically, any adhesive layer that is present between two functionally separated layers of a wound dressing, for example the backing layer and the absorbent pad, creates another interface that must be penetrated for transport, for example for transport of water vapor, through the backing layer away from the wound into the atmosphere. Any hindrance to fluid flow may impair the overall performance of the dressing.

Furthermore, if adhesives are used between layers in the manufacturing process of a wound dressing, this is typically associated with the presence of an undesirably high amount of water that needs to be removed from the dressing prior to final packaging. In processes of the art, removing residual water from adhesive is achieved in hot air ovens / convection ovens. In the process of the present invention, the use of separate equipment needed for the drying of parts of the wound dressing or the entire wound dressing, for example in energy intensive ovens can be avoided or minimized since no "constructive" adhesive is used in the disclosed process.

As discussed in aspects above, directly dispensing a silicone mixture into the "valleys" of the non-perforated parts of a support layer instead of perforating a silicone mixture as applied on a support layer, or instead of moving a silicone mixture or a silicone gel out of its original position by way of exposing the silicone mixture/gel to protruding perforating elements, results in a silicone gel layer that, when cured, is evenly spread on a perforated support layer and has openings aligning with said perforations, without, however, the silicone mixture or the silicone gel itself ever being subjected to a perforation step or a "moving out of its original place"- step.

This conceptually different aspect of creating a cured silicone gel layer on top of a perforated support layer (wherein said silicone gel layer has openings that essentially coincide with the perforations of the support layer) avoids any "pushing away" of the thermoset silicone gel material resulting in a "counterforce" that leads to a partial or complete reclosing of openings that have been created by "pushing away" silicone gel, for example by way of protruding perforating elements.

The silicone mixture dispensing process as described in aspects above not only reduces the time needed to achieve a laterally homogenous silicone gel layer with stable openings, but also avoids the formation of ring-shaped elevated areas ("donuts") around the openings at which perforation elements had protruded the silicone gel layer, since the silicone gel is never pushed outside its original position but rather is dispensed onto a support layer comprising openings which are blocked by elevated portions of the deformed template layer and thus creating suitable receptacles ("valleys") around the circumferential shape of the elevated portions of the deformed template layer.

The present disclosure also relates to the following embodiments which may be combined with each other and/or with embodiments as described above:
1. Process for the manufacture of wound dressings, said process at least comprising the steps of:
   (A) optionally feeding at least a template layer and a support layer through an ultrasonic perforation device,
      which ultrasonic perforation device comprises a plurality of perforation elements, preferably an array of perforation elements, and which device is configured to introduce perforations, in particular an array of perforations, into at least said support layer;
      wherein the perforating elements perforate the support layer, while, at the same time, deform the template layer without perforating the same;
   (B) optionally, after completion of step (A), dispensing a silicone mixture onto the support layer so that the silicone mixture at least covers the non-perforated parts of the perforated support layer on top of the deformed template layer;
   (C) optionally at least partially curing the silicone mixture from step (B) to a silicone gel to result in an at least partially cured silicone gel, optionally thereto: while the laminate from step (B) at least partially rotates on a heated rotating drum;
   (D) optionally removing the laminate from step (C) from the heated rotating drum while separating the perforated support layer with the cured silicone gel of step (C) from the deformed template layer and, if present, from other optional layer(s), and transferring said separated laminate comprising the cured silicone gel layer on the perforated support layer onto a moving belt;
   (E) continuously applying absorbent pads onto the laminate from step (D), said laminate comprising at least a perforated support layer and a silicone gel layer, wherein the absorbent pads are applied onto the side of the support layer that is not coated with silicone gel, wherein said absorbent pads are at least partially heat bonded to said side of the support layer not coated with silicone gel;
   (F) continuously feeding a backing layer onto the laminate from step (E), wherein the backing layer is applied onto the absorbent pads and the side of the support layer that is not coated with silicone gel, wherein the backing layer is at least partially heat bonded to said side of the support layer that is not coated with silicone gel.
2. The process of embodiment 1, further comprising at least one of the following steps, preferably at least further comprising steps (G) and (H) or preferably only comprising step (I):
   (G) cooling the laminate resulting from step (E) or from step (F) down to a temperature in a range of from 15°C to 40°C, preferably from 18°C to 25°C, wherein the cooled laminate is subsequently removed from the moving belt;
   (H) continuously feeding and adding a release liner onto the silicone gel layer of the laminate as removed from the moving belt in step (G),
   (I) at least partially heat bonding the backing layer onto the absorbent pad, optionally between two rotating rolls, wherein the upper roll, i.e. the roll in direct contact with the backing layer, is heated to a temperature in the range of from 120°C to 160°C.
3. The process according to any of the preceding embodiments, wherein the support layer is fed as a laminate, optionally as a laminate of polyurethane (PU) together with a reinforcing layer of polyethylene (PE).
4. The process according to any of the preceding embodiments, wherein the silicone mixture in dispensing step (B) is dispensed as an uncured two-component silicone mixture.
5. The process according to any of the preceding embodiments, wherein the curing time in step (C) is determined by adjusting one of the following, optionally by adjusting all of the following: temperature of a heated rotating drum, diameter of a heated rotating drum, speed of rotation of a rotating drum.
6. The process according to any of the preceding embodiments, wherein curing temperature, optionally as defined by the temperature on the surface of a rotating drum, is from 120°C to 150°C.
7. The process according to any of the preceding embodiments, wherein the curing time in step (C) is in the range of 20 seconds to 10 minutes, preferably from 20 seconds to 60 seconds.
8. The process according to any of the preceding embodiments, wherein the moving belt is or comprises a composite material that is compatible with temperatures of up to 150°C, preferably up to 200°C and/or that has a static coefficient of friction of below 0.15, preferably below 0.1.
9. The process according to any of the preceding embodiments, wherein the moving belt is or comprises a polyfluorinated polymer, preferably polytetrafluorethylene, particularly preferred is or comprises fiberglass reenforced polytetrafluorethylene.
10. The process according to any of the preceding embodiments, wherein the moving belt rotates continuously in the overall system
11. The process according to any of the preceding embodiments, wherein heat bonding the absorbent pads onto the support layer in step (E) is achieved by heating the moving belt from below so that the temperature of the support layer is in the range of from 100°C to 180°C, preferably from 120°C to 160°C.
12. The process according to any of the preceding embodiments, wherein a pressure is applied onto the absorbent pad as the same is brought into contact with the backing layer in step (F).
13. The process according to any of the preceding embodiments, wherein the absorbent pad comprises a hydrophilic foam, in particular a hydrophilic polyurethane-based foam, or a hydrophilic non-woven material, in particular an airlaid material comprising polyvinylalcohol polymers (PVA), polyacrylic acid fibers or derivatized cellulose polymers, in particular carboxymethyl cellulose (CMC); or any combinations of these materials.
14. Wound dressing, in particular an island wound dressing, said dressing at least comprising:
   - at least one absorbent pad;
   - a vapor permeable backing layer overlaying said absorbent pad;
   - a perforated support layer underneath said absorbent pad, wherein said backing layer and said support layer extend beyond a periphery of said absorbent pad to define a surrounding border portion along said periphery of the absorbent pad;
      further wherein the backing layer and the support layer are at least partially heat bonded together in said border region, and wherein the absorbent pad and the support layer are at least partially heat bonded together;
   - a silicone gel layer on the side of the support layer that is opposite the side that contacts the backing layer, wherein the silicone gel layer has openings that correspond to the perforations of the perforated support layer;
   - optionally a release layer covering the silicone gel layer and suitable to protect the same prior to use as a wound dressing.
15. The wound dressing according to embodiment 14, wherein the no adhesive, in particular no acrylic adhesive, is present between said backing layer and said support layer and wherein no adhesive, in particular no acrylic adhesive, is present between said absorbent pad and said support layer,
16. The wound dressing according to embodiment 14 or embodiment 15, wherein the silicone gel layer has been moulded onto the support layer, preferably has been obtained by applying a silicone mixture onto a perforated support layer and subsequently at least partially curing said silicone mixture to result in a at least partially cured, silicone gel, further preferably wherein the neither the silicone mixture nor the silicone gel has been perforated, in particular has not been exposed to any perforating elements.
17. The wound dressing according to any of embodiments 14 to 16, wherein the thickness of the silicone gel layer does not vary by more than 10%, preferably not by more than 7%, further preferably not by more than 5% across the entire lateral area of the silicone gel layer
18. The process or the wound dressing according to any of the preceding embodiments, wherein the support layer comprises a polyurethane polymer or is a layer of a polyurethane polymer.
19. The process or the wound dressing according to any of the preceding embodiments, wherein the support layer comprises a layer of a polyurethane polymer and a layer of a polyethylene polymer.
20. The process according to any of the preceding embodiments, wherein the template layer is made of thermoplastic polymer material that has a higher melting point, by at least 10 K, preferably by at least 20 K, than the polymer(s) making up the support layer.
21. The process according to embodiment 20, wherein the template layer comprises polypropylene (PP) polymer or is a layer of a polypropylene (PP) polymer.
22. The process or the wound dressing according to any of the preceding embodiments, wherein the backing layer and the perforated support layer are at least partially heat laminated together so that the adhesion strength between these two layers exceeds the peeling strength required to remove the entire wound dressing from a wound.
23. The process or the wound dressing according to any of the preceding embodiments, wherein the absorbent pad is a composite material comprising at least one foam material and at least one fiber material.

### Detailed Description of Embodiments of the Invention

In aspects, the support layer is or comprises a thermoplastic polymer layer having a melting temperature of from 100°C to 150°C, preferably from 105°C to 135°C.

Preferably, the thermoplastic polymer is also elastomeric.

Preferred polymers for the support layer are polyurethane, polyethylene, ethylene vinyl acetate, polypropylene, polyvinyl chloride, polystyrol, polyether, polyester, polyamide, polycarbonate, polyether polyamide copolymers, polyacrylate, polymethacrylate, and/or polymaleate.

In preferred aspects, the substrate layer comprises polyurethane, polyethylene or ethylene vinyl acetate. Polyurethane is particularly preferred.

In accordance with the present disclosure, any substrate layer or any combination of substrate sublayers may be used, as long as these layer(s) is/are suitable to be perforated . In particular, the substrate layer should be suitable to support a silicone mixture as applied in step (B) and the at least partially cured silicone gel resulting after step (C).

In aspects, the thickness of said substrate layer is from 5 µm to 200 µm, preferably from 10 µm to 100 µm, further preferably from 15 µm to 60 µm.

In aspects, the template layer is adapted such that it does not melt under the ultrasonic energy input as provided in the process of perforating the substrate layer. Thereby, deformed portions, or imprints, corresponding to the perforating elements may be provided without breaking the deformable film (e.g. by creating perforations therein or by complete loss of material).

In aspects, the template layer is made of thermoplastic polymer material that has a higher melting point, by at least 10 K, preferably at least 20 K, than the polymer that is used as the supporting layer and that is perforated in said perforation step.

In aspects, the melting point of the template layer is from 120 to 220°C, preferably from 130 to 190 °C

In aspects, the template layer comprises or is a material selected from polypropylene (PP), polyester, polycarbonate or polyamide. Polypropylene is particularly preferred.

In aspects, the thickness of the template layer is from 30 µm to 120 µm, preferably from 40 µm to 80 µm.

In embodiments, the backing layer preferably is or comprises polyurethane, in particular melt-blown polyurethane, which is optionally fed together with a further supporting layer, which supporting layer preferably comprises or consists of polyethylene.

Ultrasonic welding specifically for the purposes of creating perforations in a laminate is known, in principle, for example from US 4 747 895, which discloses the ultrasonic perforating of a continuously moving strip of materials (for example an adhesive-backed plastic strip). In accordance with US 4 747 895, the continuously moving strip passes through a gap defined by an ultrasonic horn and is brought in contact with a rotating drum that has perforating projections acting as an array of perforation elements.

Silicone gels as used in the laminates and in the process disclosed herein are understood to be gentle on the skin. This is because a soft silicone gel can follow the contours of the skin well thus providing a large contact surface area. Thus, although the actual adhesive force in each contact point of a silicone gel adhesive generally is less than that of a typical acrylic adhesive, the large surface area of contact achieved with a silicone gel affords a high overall adherence to the skin, whilst at the same time, the silicone gel is skin-friendly, i.e. when a silicone gel silicone gel is removed from the skin very few skin cells are coremoved due to the comparatively low adhesive force in each contact point. Therefore, the problem of skin stripping can be avoided or minimized.

In preferred aspects, the silicone gel comprises or is a two-component addition- hardening silicone gel.

In case of this preferred silicone gel. the curing process (only) starts when the two components are joined with the substrate, in particular in step (C) as outlined above.

Such a two-component silicone gel may be a chemically crosslinked silicone gel, for example a polydimethyl siloxane gel, for instance a platinum catalyzed 2-component addition hardening RTV-silicone. Examples of gels that can be used are SilGel 612 from Wacker-Chemie GmbH, Burghausen, Germany, and MED-6340 from NuSil Technology, Carpinteria, USA. Examples of silicone gel gels useful in this context are also described in GB-A-2 192 142, GB-A-2 226 780 and EP-A1-0 300 620.

In aspects, in the final product, the "grammage" (amount of silicone gel per square meter) is from 10 g/m² to 500 g/m², preferably from 15 g/m² to 200 g/m².

In accordance with the present invention, the term "*heat bonding*" or "*heat laminating*" is understood as a process in which two layers comprising a polymer, in particular a thermoplastic polymer, are heated and softened to a point so that the two layers when brought into contact, in particular also under the application of outside pressure, for example as exerted by counterrotating rolls in a continuous process, form bonds, in particular a physical bonding, between each other that remain stable once the two softened or even partially melted polymeric materials are cooled again.

It is commonly understood that such a heat bonding or heat laminating leads to an adhesion strength between the layers heat bonded or laminated together so that the adhesion strength between the layers remains intact during the entirety of the dressing's intended use, in particular also when the dressing is removed from the wound (i.e. in a "wet" state).

In embodiments, the adhesion strength between the individual layers heat laminated together, even in a wet state, is greater than the peeling strength required to remove the entire dressing from the patient.

In aspects, the material of the absorbent pads is selected from a polymeric material capable of heat-laminating, i.e. forming permanent bonding when brought in contact with another polymer layer, in particular with a thermoplastic polymer layer.

In embodiments, the absorbent pad is a hydrophilic foam, in particular a hydrophilic polyurethane-based foam, or a hydrophilic non-woven material, in particular an airlaid material based on polyvinylalcohol polymers (PVA), polyacrylic acid fibers or derivatized cellulose polymers, in particular carboxymethyl cellulose (CMC). Any combinations of the aforementioned materials are also included.

In embodiments, the absorbent material comprises or consists of superabsorbent particles and/or superabsorbent fibers.

In embodiments, the absorbent pad, in particular if said pad is or comprises gelling materials, is reinforced by non-gelling fibers or materials.

### List of reference signs:

(1) support layer
(1') perforated support layer
(2) silicone mixture layer (not cured)
(2') silicone gel (cured)
(3) absorbent pad
(4) backing layer
(5) template layer
(5') deformed template layer
(6) release liner (to be removed prior to use / protective purpose only)
(7) wound contact layer: perforated support layer + silicone gel layer (with aligned openings)
(8) arrow showing the direction of dispensing of the silicone mixture
(20), (20') sacrificial processing aid layer
(21) moving belt
(21') moving belt after delamination step
(30) sonotrode of ultrasonic device
(40) roll with perforation elements
(45), (45'), (45") heated transport rolls
(46) heating zone
(50) silicone mixture dispensing unit
(60) rotating die cutter (RDC)
(70) island dressing

## Claims

1. Process for the manufacture of wound dressings, said process comprising:
providing a laminate on a moving belt, wherein said laminate comprises at least the following layers in the following sequence of layers, starting with the layer that is in direct physical contact with said moving belt: a layer of silicone gel that has an array of openings and a perforated support layer that has an array of perforations that align with said array of openings in the silicone gel layer;
said process also comprising at least the following further steps:
(E) continuously applying absorbent pads onto said laminate comprising at least a perforated support layer and a silicone gel layer, wherein the absorbent pads are applied onto the side of the support layer that is not coated with silicone gel, wherein said absorbent pads are at least partially heat bonded to said side of the support layer not coated with silicone gel;
(F) continuously feeding a backing layer onto the laminate from step (E), wherein the backing layer is applied onto the absorbent pads and the side of the support layer that is not coated with silicone gel, wherein the backing layer is at least partially heat bonded to said side of the support layer that is not coated with silicone gel.

2. The process of claim 1, said process further comprising at least one of the following steps, preferably at least comprising steps (G) and (H) or at least comprising step (I):
(G) cooling the laminate resulting from step (E) or from step (F) down to a temperature in a range of from 15°C to 40°C, preferably from 18°C to 25°C, wherein the cooled laminate is subsequently removed from the moving belt;
(H) continuously feeding and adding a release liner onto the silicone gel layer of the laminate as removed from the moving belt in step (G),
(I) at least partially heat bonding the backing layer onto the absorbent pad, optionally between two rotating rolls, wherein the upper roll, i.e. the roll in direct contact with the backing layer, is heated to a temperature in the range of from 120°C to 160°C.

3. The process of claim 1 or claim 2, said process further comprising, prior to steps (E) and (F), at least one of the following steps, preferably all of the following steps:
(A) feeding at least a template layer and a support layer through an ultrasonic perforation device, which ultrasonic perforation device comprises a plurality of perforation elements, preferably an array of perforation elements, and which device is configured to introduce perforations, in particular an array of perforations, into at least said support layer;
wherein the perforating elements perforate the support layer, while, at the same time, deform the template layer without perforating the same;
(B) after completion of step (A), dispensing a silicone mixture onto the support layer, wherein the dispensing is performed so that the silicone mixture covers the non-perforated parts of the perforated support layer as present on top of the deformed template layer;
(C) at least partially curing the silicone mixture from step (B) to result in an at least partially cured silicone gel;
(D) separating the perforated support layer with the at least partially cured silicone gel of step (C) from the deformed template layer and, if present, from other optional layer(s), and transferring said separated laminate comprising the at least partially cured silicone gel layer on the perforated support layer onto a moving belt.

4. The process according to any of the preceding claims, wherein the moving belt is or comprises a composite material that is compatible with temperatures of up to 150°C, preferably up to 200°C and/or that has a static coefficient of friction of below 0.15, preferably below 0.1.

5. The process according to any of the preceding claims, wherein the moving belt is or comprises a polyfluorinated polymer, preferably polytetrafluorethylene, particularly preferred is or comprises fiberglass reenforced polytetrafluorethylene.

6. The process according to any of the preceding claims, wherein the moving belt rotates continuously in the overall system.

7. The process according to any of the preceding claims, wherein heat bonding the absorbent pads onto the support layer in step (E) is achieved by heating the moving belt from below so that the temperature of the support layer is in the range of from 100°C to 180°C, preferably from 120°C to 160°C.

8. The process according to any of the preceding claims, wherein a pressure is applied onto the absorbent pad as the same is brought into contact with the backing layer in step (F).

9. The process according to any of the preceding claims, wherein the absorbent pad comprises a hydrophilic foam, in particular a hydrophilic polyurethane-based foam, or a hydrophilic non-woven material, in particular an air laid material comprising polyvinyl alcohol polymers (PVA), polyacrylic acid fibers or derivatized cellulose polymers, in particular carboxymethyl cellulose (CMC); or any combinations of these materials.

10. Wound dressing, in particular an island wound dressing, said dressing at least comprising:
• at least one absorbent pad;
• a vapor permeable backing layer overlaying said absorbent pad;
• a perforated support layer underneath said absorbent pad, wherein said backing layer and said support layer extend beyond a periphery of said absorbent pad to define a surrounding border portion along said periphery of the absorbent pad;
further wherein the backing layer and the support layer are at least partially heat bonded together in said border region,
and wherein the absorbent pad and the support layer are at least partially heat bonded together;
• a silicone gel layer on the side of the support layer that is opposite the side that contacts the backing layer, wherein the silicone gel layer has openings that correspond to the perforations of the perforated support layer.

11. The wound dressing according to claim 10, wherein the no adhesive, in particular no acrylic adhesive, is present between said backing layer and said support layer and wherein no adhesive, in particular no acrylic adhesive, is present between said absorbent pad and said support layer.

12. The wound dressing according to claim 10 or claim 11, wherein the silicone gel layer has been moulded onto said support layer, preferably has been obtained by applying a silicone mixture onto a perforated support layer and subsequently at least partially curing said silicone mixture to result in a at least partially cured, silicone gel.

13. The wound dressing according to any one of claims 9 to 12, wherein the silicone gel layer has not been perforated, in particular has not been exposed to any perforating elements.

14. The wound dressing according to any of claims 9 to 13, wherein the thickness of the silicone gel layer does not vary by more than 10%, preferably not by more than 7%, further preferably not by more than 5% across the entire lateral area of the silicone gel layer

15. The process or the wound dressing according to any of the preceding claims, wherein the support layer comprises a polyurethane polymer or is a layer of a polyurethane polymer, and/or:
wherein the support layer comprises a layer of a polyurethane polymer and a layer of a polyethylene polymer, and/or:
wherein the backing layer and the perforated support layer are at least partially heat laminated together so that the adhesion strength between these two layers exceeds the peeling strength required to remove the entire wound dressing from a wound, and/or:
wherein the absorbent pad is a composite material comprising at least one foam material and at least one fiber material.
